# FASCICULE DE BREVET EUROPEEN

(11) **EP 1 022 016 B1**
(45) Date de publication et mention de la délivrance du brevet: **26.12.2007**
(21) Numéro de dépôt: 99403203.5
(22) Date de dépôt: 17.12.1999
(51) Int. Cl.: A61K 8/49, A61Q 5/06

(54) **Utilisation de composés phényl-azo-benzéniques cationiques en teinture des fibres kératiniques, compositions tinctoriales et procédés de teinture**
Verwendung von kationischen Phenylazobenzol-Verbindungen zur Färbung von Keratinfasern, Zusammensetzungen unf Färbeverfahren
Use of cationic phenylazobenzene compounds for dyeing keratin fibres, compositions and dyeing process

(30) Priorité: 19.01.1999 FR 9900506
(43) Date de publication de la demande: 26.07.2000
(73) Titulaire: L'ORÉAL, 75008 Paris (FR)
(72) Inventeur: Genet, Alain, 93600 Aulnay sous Bois (FR); Lagrange, Alain, 77700 Coupvray (FR)
(74) Mandataire: Miszputen, Laurent

(56) Documents cités:
- WO-A-99/03836
- FR-A- 1 221 122
- FR-A- 1 533 643
- FR-A- 1 565 247
- FR-A- 2 757 388
- CHEMICAL ABSTRACTS, vol. 72, no. 2, 12 janvier 1970 (1970-01-12) Columbus, Ohio, US; abstract no. 4329y, M. IIZUKA ET AL: "Cationic azo dyes" page 52; XP002115606 & JP 06 910910 A (HODOGAYA) 20 mai 1969 (1969-05-20)

## Description

L'invention a pour objet l'utilisation de composés phényl-azo-benzéniques comportant au moins un groupement cationique Z, Z étant choisi parmi des chaînes aliphatiques comportant au moins un cycle insaturé quaternisé, à titre de colorants directs dans des compositions destinées à la teinture des matières kératiniques et en particulier des compositions destinées à la teinture des fibres kératiniques humaines et notamment des cheveux, les compositions de teinture les contenant et le procédé de teinture directe correspondant.

Dans le domaine de la teinture capillaire, on recherche des colorants directs, c'est-à-dire des colorants qui, sans l'apport d'agent oxydant, sont capables de modifier par eux-mêmes la nuance naturelle des cheveux, de façon temporaire. Dans cette application, les colorants doivent satisfaire à un certain nombre de critères, et notamment engendrer des teintures reproductibles avec des nuances riches et variées permettant d'obtenir une large palette de couleurs susceptible de satisfaire le formulateur, ces teintures devant en outre être puissantes et résistantes au lavage, au frottement, à la lumière et à la transpiration.

Or, la demanderesse vient maintenant de découvrir, de façon totalement inattendue et surprenante, que des composés phényl-azo-benzéniques de formule (I) ci-après définie, comportant au moins un groupement cationique Z, Z étant des chaînes aliphatiques comportant au moins un cycle insaturé quaternisé, conviennent pour une utilisation comme colorant direct pour la teinture directe, mais en outre qu'ils permettent d'obtenir des compositions tinctoriales conduisant à des colorations puissantes et variées présentant d'excellentes propriétés de résistance aux différents traitements que peuvent subir les fibres kératiniques. Enfin, ces composés présentent une meilleure solubilité dans les milieux classiquement utilisés pour la teinture des fibres kératiniques et s'avèrent être aisément synthétisables.

Ces découvertes sont à la base de la présente invention.

L'invention a donc pour premier objet, l'utilisation, à titre de colorants directs dans des, ou pour la fabrication de, compositions tinctoriales pour fibres kératiniques humaines telles que les cheveux, de dichlorure de bis-[2-(3-méthyl-3H-imidazol-1-ium)-éthyl]-[4-(4-amino-phénylazo)-phényl]-amine et de composés phényl-azo-benzéniques de formule (I) suivante, et leurs sels d'addition avec un acide : 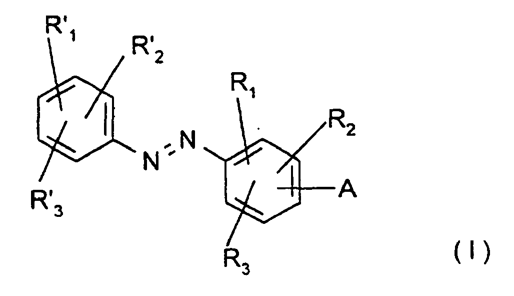 dans laquelle :
- **R₁, R₂, R₃, R'₁, R'₂,** et **R'₃,** qui peuvent être identiques ou différents, représentent un atome d'hydrogène ; un atome d'halogène ; un radical carboxy ; un radical aminosulfonyle ; un radical N-alkyl(C₁-C₆)aminosulfonyle ; un radical N,N-dialkyl(C₁-C₆)aminosulfonyle ; un radical alkyle en C₁-C₆; un radical monohydroxyalkyle en C₁-C₆ ; un radical polyhydroxyalkyle en C₂-C₆ ; un radical trifluoroalkyle en C₁-C₆ ; un radical cyano ; un radical nitro ; un groupement OR₆ défini ci-après;

**A** représente un groupement NR₄R₅ définie ci-après :
- **R₄ et R₅**, identiques ou différents, représentent un atome d'hydrogène ; un groupement Z défini ci-après ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical benzyle un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ;
- **R₆** désigne un atome d'hydrogène ; un radical alkyle en C₁-C₆; un radical monohydroxy-alkyle en C₁-C₆ ; un radical polyhydroxyalkyle en C₂-C₆ ; un radical benzyle ; un radical cyanoalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ;
- **Z** est choisi parmi les groupements cationiques insaturés de formules (II) et (III) suivantes, 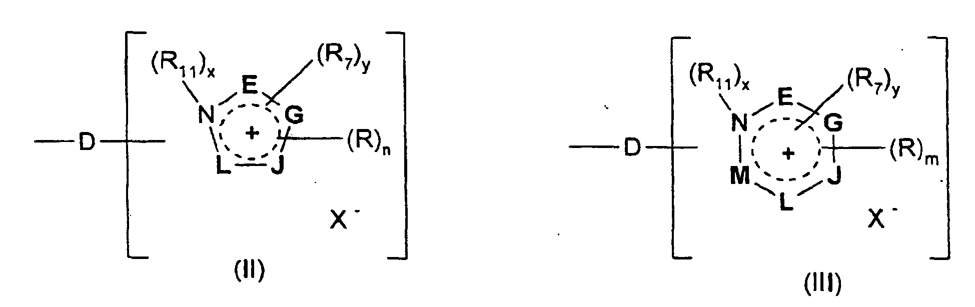 dans lesquelles :
   - D est un bras de liaison qui représente une chaîne alkyle comportant de préférence de 1 à 14 atomes de carbone, linéaire ou ramifiée, pouvant être substituée par un ou plusieurs radicaux hydroxyle ou alcoxy en C₁-C₆;
   - les sommets E, G, J, L et M, identiques ou différents, représentent un atome de carbone, d'oxygène, de soufre ou d'azote ;
   - n est un nombre entier compris entre 0 et 4 inclusivement ;
   - m est un nombre entier compris entre 0 et 5 inclusivement ;
   - les radicaux R, identiques ou différents, représentent un radical alkyle en C₁-C₆, un radical monohydroxyalkyle en C₁-C₆, un radical polyhydroxyalkyle en C₂-C₆, un radical alcoxy en C₁-C₆, un groupement ou NR"R"' dans lesquels R" et R"', identiques ou différents, représentent un radical alkyle en C₁-C₆, un radical monohydroxyalkyle en C₁-C₆ ou un radical polyhydroxyalkyle en C₂-C₆ ;
- R₇ représente un radical alkyle en C₁-C₆, un radical monohydroxyalkyle en C₁-C₆, un radical polyhydroxyalkyle en C₂-C₆, un radical benzyle.
- R₁₁ représente un radical alkyle en C₁-C₆ ; un radical monohydroxyalkyle en C₁-C₆ ; un radical polyhydroxyalkyle en C₂-C₆ ; un radical benzyle ;
- x et y sont des nombres entiers égaux à 0 ou 1 , avec les conditions suivantes :
   - dans les groupements cationiques insaturés de formule (II) :
      - lorsque x = 0, le bras de liaison D est rattaché à l'atome d'azote,
      - lorsque x = 1, le bras de liaison D est rattaché à l'un des sommets E, G, J ou L,
      - y ne peut prendre la valeur 1 que :
         1) lorsque les sommets E, G, J et L représentent simultanément un atome de carbone, et que le radical R₇ est porté par l'atome d'azote du cycle insaturé ; ou bien
         2) lorsqu'au moins un des sommets E, G, J et L représente un atome d'azote sur lequel le radical R₇ est fixé ;
   - dans les groupements cationiques insaturés de formule (III) :
      - lorsque x = 0, le bras de liaison D est rattaché à l'atome d'azote,
      - lorsque x = 1, le bras de liaison D est rattaché à l'un des sommets E, G, J, L ou M,
      - y ne peut prendre la valeur 1 que lorsqu'au moins un des sommets E, G, J, L et M représente un atome divalent, et que le radical R₇ est porté par l'atome d'azote du cycle insaturé ;
- X⁻ représente un anion monovalent ou divalent et est de préférence choisi parmi un atome d'halogène tel que le chlore, le brome, le fluor ou l'iode, un hydroxyde, un hydrogènesulfate, ou un alkyl(C₁-C₆)sulfate tel que par exemple un méthylsulfate ou un éthylsulfate ;
**étant entendu :**
- que le nombre de groupements cationiques Z de formule (II) ou (III) est au moins égal à 1 ;

Dans les formules (I), (II), et (III) ci-dessus les radicaux alkyle et alcoxy peuvent être linéaires ou ramifiés.
Les composés de formule (I) peuvent être éventuellement salifiés par des acides minéraux forts tels que HCI, HBr, H₂SO₄, ou des acides organiques tels qu'acétique, tartrique, lactique, citrique ou succinique.

Parmi les cycles des groupements insaturés Z de formule (II) ci-dessus, on peut notamment citer à titre d'exemple les cycles pyrrolique, imidazolique, pyrazolique, oxazolique, thiazolique et triazolique.

Parmi les cycles des groupements insaturés Z de formule (III) ci-dessus, on peut notamment citer à titre d'exemple les cycles pyridinique, pyrimidinique, pyrazinique, oxazinique et triazinique.

Parmi les composés de formule (I) ci-dessus, on peut notamment citer les composés suivants :
- Dichlorure de bis-[2-(3-méthyl-3H-imidazol-1-ium)-éthyl]-[4-(4-nitro-phénylazo)-phényl]-amine, monchydrate.
- Dichlorure de bis-[2-(3-méthyl-3H-imidazol-1-ium)-éthyl]-[4-(4-amino-phénylazo)-phényl]-amine.

Les composés de formule (I) conformes à l'invention peuvent être facilement obtenus, selon des méthodes bien connues de l'état de la technique pour l'obtention des amines quaternisées, par exemple :
- en un temps, par condensation d'un composé phényl-azo-benzénique comportant un radical halogénoalkyle avec un composé porteur d'un radical amine tertiaire, ou par condensation d'un composé phényl-azo-benzénique comportant un radical amine tertiaire avec un composé porteur d'un radical halogénoalkyle ;
- ou en deux temps, par condensation d'un composé phényl-azo-benzénique comportant un radical halogénoalkyle avec un composé porteur d'une amine secondaire, ou par condensation d'un composé phényl-azo-benzénique halogéné avec une amino(di-substituée)alkyleamine, suivie d'une quaternisation avec un agent alkylant.

L'étape de quaternisation est, généralement par commodité, la dernière étape de la synthèse, mais peut intervenir plus tôt dans la suite des réactions conduisant à la préparation des composés de formule (I).

L'invention a également pour objet une composition de teinture des matières kératiniques, caractérisée par le fait qu'elle comprend, dans un milieu approprié pour la teinture, une quantité efficace d'au moins un composé de formule (I) conforme à l'invention.

L'invention a aussi pour objet une composition pour la teinture directe des fibres kératiniques humaines telles que les cheveux, caractérisée par le fait qu'elle comprend, dans un milieu approprié pour la teinture, une quantité efficace d'au moins un composé de formule (I) conforme à l'invention.

Le ou les composés phényl-azo-benzéniques cationiques de formule (I) conformes à l'invention et/ou le ou leurs sels d'addition avec un acide représentent de préférence de 0,005 à 12 % en poids environ du poids total de la composition tinctoriale, et encore plus préférentiellement de 0,05 à 6 % en poids environ de ce poids.

Les composés de formule (I) conformes à l'invention peuvent également servir, dans les procédés bien connus de teinture d'oxydation, utilisant des colorants d'oxydation (précurseurs de colorants d'oxydation et éventuellement des coupleurs), à nuancer ou enrichir de reflets les teintures obtenues avec les colorants d'oxydation.

La composition tinctoriale selon l'invention peut encore contenir, pour élargir la palette de nuances et obtenir des teintes variées, outre les composés phényl-azo-benzéniques cationiques de formule (I) selon l'invention, d'autres colorants directs classiquement utilisés, et notamment, des colorants nitrés benzéniques, comme les nitrophénylènediamines, les nitrodiphénylamines, les nitroanilines, les éthers de phénol nitrés ou les nitrophénols, des nitropyridines, des colorants anthraquinoniques, des colorants mono- ou di-azoïques, triarylméthaniques, aziniques, acridiniques et xanthéniques, ou encore des colorants métallifères.

La proportion de tous ces autres colorants directs d'addition peut varier entre 0,5 et 10% en poids environ par rapport au poids total de la composition tinctoriale.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

On peut également ajouter à la composition selon l'invention des amides gras tels que les mono- et di-éthanolamides des acides dérivés du coprah, de l'acide laurique ou de l'acide oléïque, à des concentrations comprises entre environ 0,05 et 10% en poids.

On peut encore ajouter à la composition selon l'invention des agents tensio-actifs bien connus de l'état de la technique et de type anionique, cationique, non-ionique, amphotère, zwittérionique ou leurs mélanges, de préférence en une proportion comprise entre environ 0,1 et 50% en poids et avantageusement entre environ 1 et 20% en poids par rapport au poids total de la composition.
On peut également utiliser des agents épaississants dans une proportion allant d'environ 0,2 à 5%.
Ladite composition tinctoriale peut contenir en outre divers adjuvants usuels tels que des agents anti-oxydants, des parfums, des agents séquestrants, des agents dispersants, des agents de conditionnement du cheveu, des agents conservateurs, des agents opacifiants, ainsi que tout autre adjuvant utilisé habituellement en teinture des matières kératiniques.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (V) suivante : 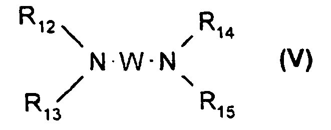 dans laquelle W est un reste propylène éventuellement substitué par un groupement hydroxyle ou un radical alkyle en C₁-C₆ ; R₁₂, R₁₃, R₁₄ et R₁₅, identiques ou différents, représentent un atome d'hydrogène, un radical alkyle en C₁-C₆ ou hydroxyalkyle en C₁-C₆.

La composition tinctoriale selon l'invention peut se présenter sous des formes diverses, telles que sous forme de liquides, de crèmes, de gels, ou sous toute autre forme appropriée pour réaliser une teinture des matières kératiniques, et notamment des cheveux humains. En particulier, elle peut être conditionnée sous pression en flacon aérosol en présence d'un agent propulseur et former une mousse.

Un autre objet de l'invention porte sur un procédé de teinture des fibres kératiniques et en particulier des fibres kératiniques humaines telles que les cheveux, par coloration directe, consistant à laisser agir une composition renfermant au moins un composé phényl-azo-benzénique cationique de formule (I) sur les fibres kératiniques sèches ou humides.
On peut utiliser la composition selon l'invention en tant que composition non rincée, c'est-à-dire qu'après application de la composition sur les fibres, on sèche sans rinçage intermédiaire.
Dans les autres modes d'application, après application de la composition sur les fibres pendant un temps de pose variant entre 3 et 60 minutes environ, de préférence entre 5 et 45 minutes environ, on rince, éventuellement on lave, puis on rince à nouveau, et on sèche.

Des exemples concrets et non limitatifs illustrant l'invention vont maintenant être donnés.

### EXEMPLE DE PREPARATION

### Exemple 1 :

Dichlorure de bis-[2-(3-méthyl-3H-imidazol-1-ium)-éthyl]-[4-(4-nitro-phénylazo)-phényl]-amine, monohydrate.

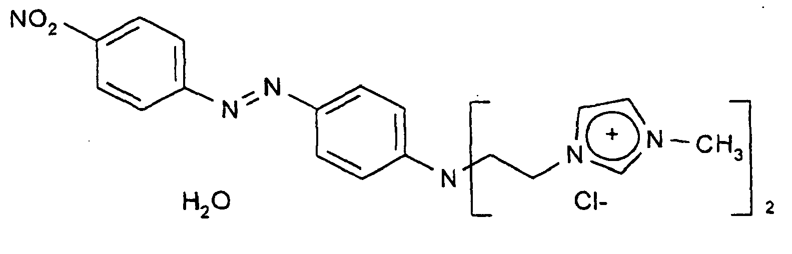

On a porté au reflux pendant 8 heures un mélange de 21,2g (0,057 mole) de bis-(2-chloro-éthyl)-[4-(4-nitro-phénylazo)-phényl]-amine (RN 66710-74-5) et de 37,4g (0,456 mole) de 1-méthyl-1 H-imidazole (RN 616-47-7) dans 50 ml de toluène.
La solution rouge est devenue une suspension (précipité cristallisé).
On a ajouté 40 ml d'isobutanol et à nouveau 37,4g (0,456 mole) de 1-méthyl-1H-imidazole. Le reflux a été prolongé pendant 8 heures.
Le précipité cristallisé a été essoré, lavé avec du toluène et purifié par recristallisation de l'éthanol à 96° au reflux.
On a obtenu 26,8g de cristaux rouges de dichlorure de bis-[2-(3-méthyl-3H-imidazol-1-ium)-éthyl]-[4-(4-nitro-phénylazo)-phényl]-amine, monohydrate fondant avec décomposition à une température supérieure à 260°C (Kofler).

L'analyse élémentaire pour C₂₄ H₂₈ N₈ O₂Cl₂ + H₂O était :

| **%** | **C** | **H** | **N** | **O** | **Cl** |
|---|---|---|---|---|---|
| théorie | 52,46 | 5,50 | 20,39 | 8,74 | 12,90 |
| trouvé | 52,60 | 5,47 | 20,50 | 8,55 | 13,10 |

### EXEMPLES DE COMPOSITIONS TINCTORIALES

### EXEMPLES 1 à 2 :

On a préparé les 2 compositions de teinture directe réunies dans le tableau suivant *(toutes teneurs exprimées en grammes - M.A. désigne Matière Active):*

| | Exemple 1 | Exemple 2 |
|---|---|---|
| colorant de l'exemple 1 | 0,549 | |
| colorant de l'exemple 1 | | 0,549 |
| Hydroxyéthylcellulose vendue sous la dénomination NATROSOL 250 MR par la société Aqualon | 0,77 | 0,77 |
| Alcool benzylique | 4 | 4 |
| Polyéthylèneglycol à 300 oxyde d'éthylène | 12 | 12 |
| Alkyl(C8-C10) polyglucoside en solution aqueuse à 60% M.A. vendu sous la dénomination ORAMIX CG 110 par la société Seppic | 6 M.A. | 6 M.A. |
| Tampon phosphate pH 7 q.s.p. | 100 | |
| Tampon phosphate pH 9 (acide borique/chlorure de potassium/hydroxyde de sodium q.s.p. | | 100 |

On a appliqué chacune des compositions ci-dessus sur des mèches de cheveux gris naturels ou permanentés à 90% de blancs et on a laissé poser pendant 20 minutes. Après rinçage à l'eau courante et séchage, les cheveux ont été teints dans une nuance qui est exprimée dans le tableau ci-dessous.

| | |
|---|---|
| Composition de l'exemple 1 | Cuivré |
| Composition de l'exemple 2 | Cuivré |

## Revendications

1. Utilisation à titre de colorants directs dans des compositions tinctoriales pour kératiniques humaines, de dichlorure de bis-[2-(3-méthyl-3H-imidazol-1-ium)-éthyl]-[4-(4-amino-phénylazorphényl]-amine et de composés de formule 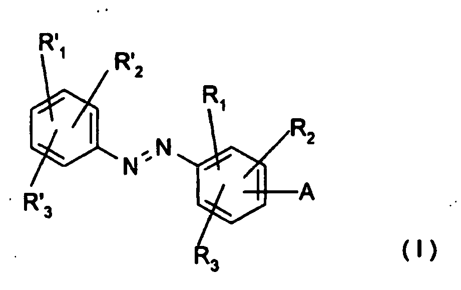 dans laquelle :
• **R₁, R₂, R₃, R'₁, R'₂**, et **R'₃,** qui peuvent être identiques ou différents, représentent un atome d'hydrogène ; un atome d'halogène ; un radical carboxy ; un radical aminosulfonyle ; un radical N-alkyl(C₁-C₆)aminosulfonyle ; un radical N,N-dialkyl(C₁-C₆)aminosulfonyle ; un radical alkyle en C₁-C₆ ; un radical monohydroxyalkyle en C₁-C₆ : un radical polyhydroxyalkyle en C₂-C₆ ; un radical trifluoroalkyle en C₁-C₆ ; un radical cyano ; un radical nitro ; un groupement OR₆ défini ci-après ;
**A** représente un groupement NR₄R₅ définis ci-après;
• **R₄ et R₅,** identiques ou différents, représentent un atome d'hydrogène ; un groupement Z défini ci-après ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical benzyle ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ;
• **R₆** désigne un atome d'hydrogène ; un radical alkyle en C₁-C₆; un radical monohydroxy-alkyle en C₁-C₆ ; un radical polyhydroxyalkyle en C₂-C₆ ; un radical benzyle ; un radical cyanoalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ;
• **Z** est choisi parmi les groupements cationiques insaturés de formules (II) et (III) suivantes: 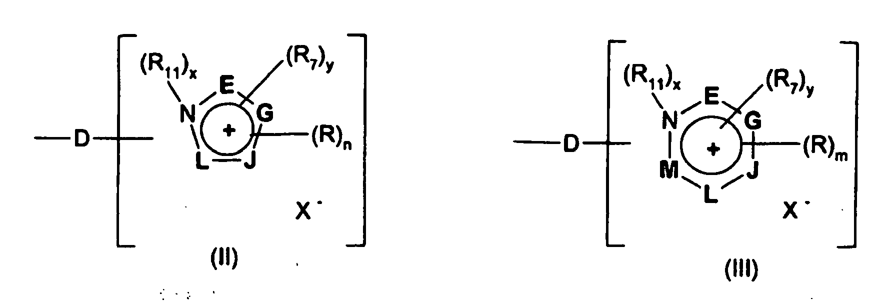
dans lesquelles :
• D est un bras de liaison qui représente une chaîne alkyle comportant de préférence de 1 à 14 atomes de carbone, linéaire ou ramifiée pouvant être substituée par un ou plusieurs radicaux hydroxyle ou alcoxy en C₁-C₆ ;
• les sommets E, G, J, L et M, identiques ou différents, représentent un atome de carbone, d'oxygène, de soufre ou d'azote ;
• n est un nombre entier compris entre 0 et 4 inclusivement ;
• m est un nombre entier compris entre 0 et 5 inclusivement ;
• les radicaux R, identiques ou différents, représentent un radical alkyle en C₁-C₆, un radical monohydroxyalkyle en C₁-C₆, un radical polyhydroxyalkyle en C₂-C₆, un radical alcoxy en C₁-C₆, un groupement NR"R"' dans lesquels R" et R"', identiques ou différents, représentent un radical alkyle en C₁-C₆, un radical monohydroxyalkyle en C₁-C₆ ou un radical polyhydroxyalkyle en C₂-C₆ ;
• R₇ représente un radical alkyle en C₁-C₆, un radical monohydroxyalkyle en C₁-C₆, un radical polyhydroxyalkyle en C₂-C₆, un radical benzyle;
• R₁₁ représente un radical alkyle en C₁-C₆ ; un radical monohydroxyalkyle en C₁-C₆ ; un radical polyhydroxyalkyle en C₂-C₆ ; benzyle ;
• x et y sont des nombres entiers égaux à 0 ou 1 , avec les conditions suivantes :
- dans les groupements cationiques insaturés de formule (II)
- lorsque x = 0, le bras de liaison D est rattaché à l'atome d'azote,
- lorsque x = 1, le bras de liaison D est rattaché à l'un des sommets E, G, J ou L,
- y ne peut prendre la valeur 1 que :
1) lorsque les sommets E, G, J et L représentent simultanément un atome de carbone, et que le radical R₇ est porté par l'atome d'azote du cycle insaturé ; ou bien
2) lorsqu'au moins un des sommets E, G, J et L représente un atome d'azote sur lequel le radical R₇ est fixé ;
- dans les groupements cationiques insaturés de formule (III) :
- lorsque x = 0, le bras de liaison D est rattaché à l'atome d'azote,
- lorsque x = 1, le bras de liaison D est rattaché à l'un des sommets E, G, J, L ou M,
- y ne peut prendre la valeur 1 que lorsqu'au moins un des sommets E, G, J, L et M représente un atome divalent, et que le radical R₇ est porté par l'atome d'azote du cycle insaturé ;
• **X⁻** représente un anion monovalent ou divalent ;
**étant entendu :**
- que le nombre de groupements cationiques Z de formule (II) ou (III) est au moins égal à 1 ;

2. Utilisation selon la revendication 1, **caractérisée par le fait que** les cycles des groupements insaturés Z de formule (II) sont choisis parmi les cycles pyrrolique, imidazolique, pyrazolique, oxazolique, thiazolique et triazolique.

3. Utilisation selon la revendication 1, **caractérisée par le fait que** les cycles des groupements insaturés Z de formule (III) sont choisis parmi les cycles pyridinique, pyrimidinique, pyrazinique, oxazinique et triazinique.

4. Utilisation selon l'une quelconque des revendications précédentes, **caractérisée par le fait que** X est choisi parmi un atome d'halogène, un hydroxyde, un hydrogènesulfate, ou un alkyl(C₁-C₆)sulfate.

5. Utilisation selon l'une quelconque des revendications précédentes, **caractérisée par le fait que** les composés de formule (I) sont choisis parmi les suivants :
- Dichlorure de bis-[2-(3-méthyl-3H-imidazol-1-ium)-éthyl]-[4-(4-nitro-phénylazo)-phényl]-amine, monohydrate.
- Dichlorure de bis-[2-(3-méthyl-3H-imidazol-1-ium)-éthyl]-[4-(4-amino-phénylazo)-phényl]-amine.

6. Utilisation selon l'une quelconque des revendications précédentes, **caractérisée par le fait que** les sels d'addition avec un acide des composés de formule (I) sont choisis parmi les chlorhydrates, les bromhydrates, les sulfates, les citrates, les succinates, les tartrates, les lactates et les acétates.

7. Composition tinctoriale pour matières kératiniques humaines, **caractérisée par le fait qu'**elle comprend, dans un milieu approprié pour la teinture, une quantité efficace d'au moins un composé de formule (I) défini à l'une quelconque des revendications 1-5 et des agents tensioactifs.

8. Composition selon la revendication 7, 9, **caractérisée par le fait qu'**elle a un pH compris entre 3 et 12.

9. Composition selon l'une quelconque des revendications 7 ou 8, **caractérisée par le fait que** les composés de formule (I) sont présents dans une concentration allant de 0,005 à 12 % en poids par rapport au poids total de la composition.

10. Composition selon la revendication 9, **caractérisée par le fait que** les composés de formule (I) sont présents dans une concentration allant de 0,05 à 6 % en poids par rapport au poids total de la composition.

11. Composition selon l'une quelconque des revendications 7 à 10, **caractérisée par le fait que** le milieu approprié pour la teinture est un milieu aqueux constitué par de l'eau et/ou des solvants organiques, dans des proportions comprises entre 1 et 40% en poids par rapport au poids total de la composition.

12. Procédé de teinture des fibres kératiniques humaines par coloration directe, consistant à laisser agir une composition tinctoriale telle que définie à l'une quelconque des revendications 9 à 11, sur les fibres sèches ou humides.

13. Procédé de teinture
**caractérisé par le fait qu'**on applique une composition tinctoriale telle que définie à l'une quelconque des revendications 7 à 11, sur les fibres kératiniques sèches ou humides, et qu'on sèche ces fibres sans rinçage intermédiaire.

14. Procédé de teinture
**caractérisé par le fait qu'**on applique une composition tinctoriale telle que définie à l'une quelconque des revendications 7 à 11 sur les fibres kératiniques sèches ou humides, et qu'après avoir éventuellement laissé agir la composition sur les fibres pendant un temps de pose allant de 3 à 60 minutes, on rince les fibres, on les lave éventuellement, on les rince à nouveau puis on les sèche.

## Claims

1. Use, as direct dyes, in dye compositions for human keratin fibres, of bis[2-(3-methyl-3H-imidazol-1-ium)ethyl][4-(4-aminophenylazo)phenyl]amine dichloride and of compounds of formula (I) below, and the addition salts thereof with an acid: 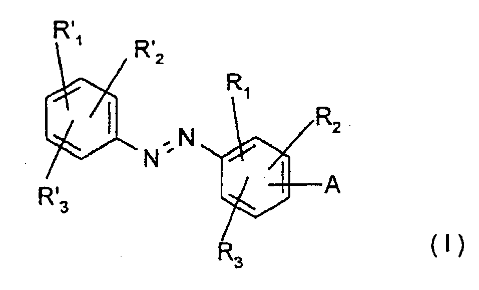 in which:
• **R₁, R₂, R₃, R'₁, R'₂** and **R'₃,** which may be identical or different, represent a hydrogen atom; a halogen atom; a carboxyl radical; an aminosulfonyl radical; a C₁-C₆ N-alkylaminosulfonyl radical; a C₁-C₆ N,N-dialkylaminosulfonyl radical; a C₁-C₆ alkyl radical; a C₁-C₆ monohydroxyalkyl radical; a C₂-C₆ polyhydroxyalkyl radical; a C₁-C₆ trifluoroalkyl radical; a cyano radical; a nitro radical; or a group OR₆ defined below;
A represents a group NR₄R₅ defined below;
• **R₄ and R₅,** which may be identical or different, represent a hydrogen atom; a group Z defined below; a C₁-C₆ alkyl radical; a C₁-C₆ monohydroxyalkyl radical; a C₂-C₆ polyhydroxyalkyl radical; a (C₁-C₆) alkoxy (C₁-C₆) alkyl radical; a benzyl radical; a cyano (C₁-C₆) alkyl radical; a carbamyl (C₁-C₆) alkyl radical; an N- (C₁-C₆)alkylcarbamyl(C₁-C₆)alkyl radical; an N,N-di(C₁-C₆)alkylcarbamyl(C₁-C₆)alkyl radical; a C₁-C₆ aminosulfonylalkyl radical; an N-(C₁-C₆)alkylaminosulfonyl (C₁-C₆)alkyl radical; or an N,N-di(C₁-C₆)alkylaminosulfonyl(C₁-C₆)alkyl radical;
• **R₆** denotes a hydrogen atom; a C₁-C₆ alkyl radical; a C₁-C₆ monohydroxyalkyl radical; a C₂-C₆ polyhydroxyalkyl radical; a benzyl radical; a cyano(C₁-C₆)alkyl radical; an N- (C₁-C₆)alkylcarbamyl(C₁-C₆)alkyl radical; or an N,N-di (C₁-C₆)alkylcarbamyl(C₁-C₆)alkyl radical;
• Z is chosen from the unsaturated cationic groups of formulae (II) and (III) below: 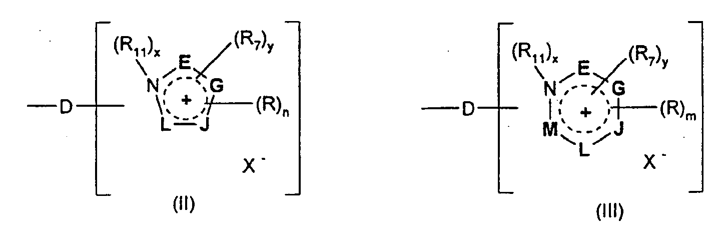
in which:
• D is a linker arm which represents a linear or branched alkyl chain preferably containing from 1 to 14 carbon atoms, which can be substituted with one or more hydroxyl or C₁-C₆ alkoxy radicals;
• the ring members E, G, J, L and M, which may be identical or different, represent a carbon, oxygen, sulfur or nitrogen atom;
• n is an integer between 0 and 4 inclusive;
• m is an integer between 0 and 5 inclusive;
• the radicals R, which may be identical or different, represent a C₁-C₆ alkyl radical, a C₁-C₆ monohydroxyalkyl radical, a C₂-C₆ polyhydroxyalkyl radical, a C₁-C₆ alkoxy radical, or a group NR"R'" in which R" and R"', which may be identical or different, represent a C₁-C₆ alkyl radical, a C₁-C₆ monohydroxyalkyl radical or a C₂-C₆ polyhydroxyalkyl radical;
• R₇ represents a C₁-C₆ alkyl radical, a C₁-C₆ monohydroxyalkyl radical, a C₂-C₆ polyhydroxyalkyl radical, or a benzyl radical;
• R₁₁ represents a C₁-C₆ alkyl radical; a C₁-C₆ monohydroxyalkyl radical; a C₂-C₆ polyhydroxyalkyl radical; or a benzyl radical;
• x and y are integers equal to 0 or 1, with the following conditions:
- in the unsaturated cationic groups of formula (II) :
- when x = 0, the linker arm D is attached to the nitrogen atom,
- when x = 1, the linker arm D is attached to one of the ring members E, G, J or L,
- y can take the value 1 only:
1) when the ring members E, G, J and L simultaneously represent a carbon atom and when the radical R₇ is borne by the nitrogen atom of the unsaturated ring; or alternatively
2) when at least one of the ring members E, G, J and L represents a nitrogen atom to which the radical R₇ is attached;
- in the unsaturated cationic groups of formula (III) :
- when x = 0, the linker arm D is attached to the nitrogen atom,
- when x = 1, the linker arm D is attached to one of the ring members E, G, J, L or M,
- y can take the value 1 only when at least one of the ring members E, G, J, L and M represents a divalent atom and when the radical R₇ is borne by the nitrogen atom of the unsaturated ring;
• X⁻ represents a monovalent or divalent anion; **it being understood:**
- that the number of cationic groups Z of formula (II) or (III) is at least equal to 1.

2. Use according to Claim 1, **characterized in that** the rings of the unsaturated groups Z of formula (II) are chosen from pyrrole, imidazole, pyrazole, oxazole, thiazole and triazole rings.

3. Use according to Claim 1, **characterized in that** the rings of the unsaturated groups Z of formula (III) are chosen from pyridine, pyrimidine, pyrazine, oxazine and triazine rings.

4. Use according to any one of the preceding claims,
**characterized in that** X⁻ is chosen from a halogen atom, a hydroxide, a hydrogen sulfate or a C₁-C₆ alkyl sulfate.

5. Use according to any one of the preceding claims,
**characterized in that** the compounds of formula (I) are chosen from the following:
- bis[2-(3-methyl-3H-imidazol-1-ium)ethyl][4-(4-nitrophenylazo)phenyl]amine dichloride monohydrate;
- bis[2-(3-methyl-3H-imidazol-1-ium)ethyl][4- (4-aminophenylazo)phenyl]amine dichloride.

6. Use according to any one of the preceding claims, **characterized in that** the addition salts with an acid of the compounds of formula (I) are chosen from the hydrochlorides, hydrobromides, sulfates, citrates, succinates, tartrates, lactates and acetates.

7. Dye composition for human keratin substances, **characterized in that** it comprises, in a medium which is suitable for dyeing, an effective amount of at least one compound of formula (I) defined in any one of Claims 1-5 and surfactants.

8. Composition according to Claim 7, **characterized in that** it has a pH of between 3 and 12.

9. Composition according to either of Claims 7 and 8, **characterized in that** the compounds of formula (I) are present in a concentration ranging from 0.005 to 12% by weight relative to the total weight of the composition.

10. Composition according to Claim 9, **characterized in that** the compounds of formula (I) are present in a concentration ranging from 0.05 to 6% by weight relative to the total weight of the composition.

11. Composition according to any one of Claims 7 to 10, **characterized in that** the medium which is suitable for dyeing is an aqueous medium consisting of water and/or of organic solvents, in proportions of between 1 and 40% by weight relative to the total weight of the composition.

12. Process for dyeing human keratin fibres by direct dyeing, consisting in leaving a dye composition as defined in any one of Claims 7 to 11 to act on the wet or dry fibres.

13. Dyeing process, **characterized in that** a dye composition as defined in any one of Claims 7 to 11 is applied to the wet or dry keratin fibres and these fibres are dried without intermediate rinsing.

14. Dyeing process, **characterized in that** a dye composition as defined in any one of Claims 7 to 11 is applied to the wet or dry keratin fibres and **in that**, after the composition has optionally been left to act on the fibres for an exposure time ranging from 3 to 60 minutes, the fibres are rinsed, optionally washed, rinsed again and then dried.

## Patentansprüche

1. Verwendung von Bis-[2-(3-methyl-3H-imidazol-1-ium)-ethyl]-[4-(4-aminophenylazo)-phenyl]amindichlorid und Verbindungen der folgenden Formel (I) und deren Additionssalzen mit einer Säure als Direktfarbstoffe in Farbmittelzusammensetzungen für menschliche Keratinfasern: 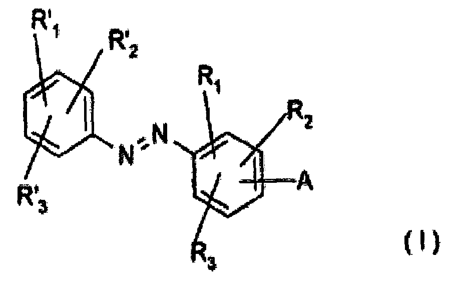 worin bedeuten:
• **R₁, R₂, R₃, R'₁, R'₂ und R'₃,** die gleich oder verschieden sein können, ein Wasserstoffatom; ein Halogenatom; eine Carboxygruppe; eine Aminosulfonylgruppe; eine N-Alkyl(C₁₋₆)-aminosulfonylgruppe; eine N,N-Dialkyl(C₁₋₆)aminosulfonylgruppe; eine C₁₋₆-Alkylgruppe; eine C₁₋₆-Monohydroxyalkylgruppe; eine C₂₋₆-Polyhydroxyalkylgruppe; eine C₁₋₆-Trifluoralkylgruppe; eine Cyanogruppe; eine Nitrogruppe; eine nachstehend definierte Gruppe OR_{6;}
• **A** eine nachstehend definierte Gruppe NR₄R₅;
• **R₄** und **R₅**, die gleich oder verschieden sind, ein Wasserstoffatom; eine nachstehend definierte Gruppe Z; C₁₋₆-Alkyl; C₁₋₆-Monohydroxyalkyl; C₂₋₆-Polyhydroxyalkyl; Alkoxy(C₁₋₆)-alkyl(C₁₋₆); Benzyl; Cyanoalkyl(C₁₋₆); Carbamoylalkyl(C₁₋₆); N-Alkyl(C₁₋₆)carbamoylalkyl(C₁₋₆); N,N-Dialkyl(C₁₋₆)carbamoylalkyl(C₁₋₆); Aminosulfonylalkyl(C₁₋₆); N-Alkyl(C₁₋₆)aminosulfonylalkyl(C₁₋₆); N,N-Dialkyl(C₁₋₆)aminosulfonylalkyl(C₁₋₆);
• **R₆** ein Wasserstoffatom; C₁₋₆-Alkyl; C₁₋₆-Monohydroxyalkyl; C₂₋₆-Polyhydroxyalkyl; Benzyl; Cyanoalkyl(C₁₋₆); N-Alkyl-(C₁₋₆)carbamoylalkyl(C₁₋₆); N,N-Dialkyl(C₁₋₆)carbamoylalkyl(C₁₋₆);
• **Z** ist unter den ungesättigten kationischen Gruppen der folgenden Formeln (II) und (III) ausgewählt: 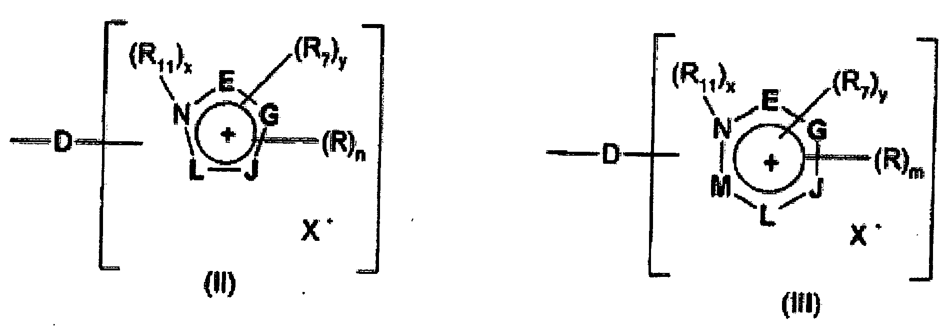
worin bedeuten:
• D eine Verbindungsgruppe, bei der es sich um eine Alkylkette mit vorzugsweise 1 bis 14 Kohlenstoffatomen handelt, die geradkettig oder verzweigt ist und mit einer oder mehreren Hydroxygruppen oder C₁₋₆-Alkoxygruppen substituiert sein kann;
• die Ringbestandteile E, G, J, L und M, die gleich oder verschieden sind, ein Kohlenstoffatom, ein Sauerstoffatom, ein Schwefelatom oder ein Stickstoffatom;
• n 0 oder eine ganze Zahl von 1 bis 4;
• m 0 oder eine ganze Zahl von 1 bis 5;
• die Gruppen R, die gleich oder verschieden sind, C₁₋₆-Alkyl, C₁₋₆-Monohydroxyalkyl, C₂₋₆-Polyhydroxyalkyl, C₁₋₆-Alkoxy, eine Gruppe NR"R"', worin R" und R"', die gleich oder verschieden sind, eine C₁₋₆-Alkylgruppe, eine C₁₋₆-Monohydroxyalkylgruppe oder eine C₂₋₆-Polyhydroxyalkylgruppe bedeuten;
• R₇ C₁₋₆-Alkyl, C₁₋₆-Monohydroxyalkyl, C₂₋₆-Polyhydroxyalkyl; Benzyl;
• R₁₁ C₁₋₆-Alkyl; C₁₋₆-Monohydroxyalkyl; C₂₋₆-Polyhydroxyalkyl; Benzyl;
• x und y 0 oder 1, mit den folgenden Maßgaben:
- in den ungesättigten kationischen Gruppen der Formel (II) :
- wenn x = 0, ist die Verbindungsgruppe D an das Stickstoffatom gebunden,
- wenn x = 1, ist die Verbindungsgruppe D an einen der Ringbestandteile E, G, J oder L gebunden,
- y kann nur den Wert 1 annehmen, wenn:
1) die Ringbestandteile E, G, J und L gleichzeitig ein Kohlenstoffatom bedeuten und die Gruppe R₇ von dem Stickstoffatom des ungesättigten Rings getragen wird; oder
2) mindestens ein Ringbestandteil E, G, J und L ein Stickstoffatom bedeutet, an das die Gruppe R₇ gebunden ist;
- in den ungesättigten kationischen Gruppen der Formel (III):
- wenn x = 0, ist die Verbindungsgruppe D an das Stickstoffatom gebunden,
- wenn x = 1, ist die Verbindungsgruppe D an einen der Ringbestandteile E, G, J, L oder M gebunden,
- y kann nur den Wert 1 annehmen, wenn mindestens einer der Ringbestandteile E, G, J, L und M ein zweiwertiges Atom bedeutet und die Gruppe R₇ von dem Stickstoffatom des ungesättigten Rings getragen wird.
• **X⁻** ein einwertiges oder zweiwertiges Anion;
**mit der Maßgabe,**
- dass die Anzahl der kationischen Gruppen Z der Formel (II) oder (III) mindestens 1 beträgt.

2. Verwendung nach Anspruch 1, **dadurch gekennzeichnet, dass** die Ringe der ungesättigten Gruppen Z der Formel (II) unter Pyrrol, Imidazol, Pyrazol, Oxazol, Thiazol und Triazol ausgewählt sind.

3. Verwendung nach Anspruch 1, **dadurch gekennzeichnet, dass** die Ringe der ungesättigten Gruppen Z der Formel (III) unter Pyridin, Pyrimidin, Pyrazin, Oxazin und Triazin ausgewählt sind.

4. Verwendung nach einem der vorhergehenden Ansprüche, **dadurch gekennzeichnet, dass** X- unter einem Halogenatom, einem Hydroxid, einem Hydrogensulfat oder einem Alkyl(C₁₋₆)sulfat ausgewählt ist.

5. Verwendung nach einem der vorhergehenden Ansprüche, **dadurch gekennzeichnet, dass** die Verbindungen der Formel (I) unter den folgenden Verbindungen ausgewählt ist:
- Bis-[2-(3-methyl-3H-imidazol-1-ium)-ethyl]-[4-(4-nitrophenylazo)-phenyl]-amindichlorid, Monohydrat.
- Bis-[2-(3-methyl-3H-imidazol-1-ium)-ethyl]-[4-(4-aminophenylazo)-phenyl]amindichlorid.

6. Verwendung nach einem der vorhergehenden Ansprüche, **dadurch gekennzeichnet, dass** die Additionssalze der Verbindungen der Formel (I) mit einer Säure unter den Hydrochloriden, Hydrobromiden, Sulfaten, Citraten, Succinaten, Tartraten, Lactaten und Acetaten ausgewählt sind.

7. Farbmittelzusammensetzung für menschliche Keratinsubstanzen, **dadurch gekennzeichnet, dass** sie in einem zum Färben geeigneten Medium eine wirksame Menge mindestens einer Verbindung der Formel (I), die in einem der Ansprüche 1 bis 5 definiert ist, und grenzflächenaktive Stoffe enthält.

8. Zusammensetzung nach Anspruch 7, **dadurch gekennzeichnet, dass** sie einen pH-Wert von 3 bis 12 aufweist.

9. Zusammensetzung nach einem der Ansprüche 7 oder 8, **dadurch gekennzeichnet, dass** die Verbindungen der Formel (I) in einer Konzentration von 0,005 bis 12 Gew.-%, bezogen auf das Gesamtgewicht der Zusammensetzung, enthalten sind.

10. Zusammensetzung nach Anspruch 9, **dadurch gekennzeichnet, dass** die Verbindungen der Formel (I) in einer Konzentration von 0,05 bis 6 Gew.-%, bezogen auf das Gesamtgewicht der Zusammensetzung, enthalten sind.

11. Zusammensetzung nach einem der Ansprüche 7 bis 10, **dadurch gekennzeichnet, dass** das zum Färben geeignete Medium ein wässriges Medium ist, das aus Wasser und/ oder organischen Lösungsmitteln in Mengenanteilen von 1 bis 40 Gew.-%, bezogen auf das Gesamtgewicht der Zusammensetzung, besteht.

12. Verfahren zum Färben von menschlichen Keratinsubstanzen durch Direktfärbung, das darin besteht, eine Farbmittelzusammensetzung nach einem der Ansprüche 7 bis 11 auf die trockenen oder feuchten Fasern einwirken zu lassen.

13. Verfahren zum Färben, **dadurch gekennzeichnet, dass** eine Farbmittelzusammensetzung nach einem der Ansprüche 7 bis 11 auf die trockenen oder feuchten Keratinsubstanzen aufgebracht wird und die Fasern ohne zwischenzeitliches Spülen getrocknet werden.

14. Verfahren zum Färben, **dadurch gekennzeichnet, dass** eine Farbmittelzusammensetzung nach einem der Ansprüche 7 bis 11 auf die trockenen oder feuchten Keratinfasern aufgebracht wird und die Fasern, nachdem die Zusammensetzung gegebenenfalls auf die Fasern während einer Einwirkzeit von 3 bis 60 Minuten einwirken gelassen wurde, gespült, gegebenenfalls gewaschen, nochmals gespült und dann getrocknet werden.
